# EUROPEAN PATENT SPECIFICATION

(11) **EP 4 193 955 B1**
(45) Date of publication and mention of the grant of the patent: **18.12.2024**
(21) Application number: 20952371.1
(22) Date of filing: 01.09.2020
(51) Int. Cl.: A61B 34/00, A61B 34/30

(54) **SURGICAL TOOL**
CHIRURGISCHES WERKZEUG
INSTRUMENT CHIRURGICAL

(43) Date of publication of application: 14.06.2023
(73) Proprietor: RIVERFIELD Inc., 1070052 Tokyo (JP)
(72) Inventor: SHINDO, Koki, Tokyo 107-0052 (JP)
(74) Representative: adares Patent- und Rechtsanwälte Reininger & Partner GmbB
(86) International application number: PCT/JP2020/033087
(87) International publication number: WO 2022/049631

(56) References cited:
- WO-A1-2017/208320
- WO-A1-2019/244799
- CN-A- 110 353 807
- CN-A- 110 353 807
- JP-A- 2019 530 530
- JP-A- H04 329 939
- JP-A- H04 329 939
- JP-A- H11 104 137
- JP-A- H11 104 137
- US-A1- 2019 231 465

## Description

### TECHNICAL FIELD

The present disclosure relates to a surgical tool.

### BACKGROUND ART

Surgical tools used for medical procedures such as endoscopic surgeries have been known. Such a surgical tool includes a treatment portion disposed, for example, on a leading end of the surgical tool, and an operation unit for an operator to manipulate. The manipulation on the operation unit is transmitted to the treatment portion through a wire, which causes the treatment portion to move in response to the manipulation by the operator (see, Patent Document 1).

### PRIOR ART DOCUMENTS

### PATENT DOCUMENTS

Patent Document 1: Published Japanese Translation of PCT International Publication for Patent Application No. 2016-512961
Patent Document 2: CN 110 353 807 A
Patent Document 3: JP H11 104137 A
Patent Document 4: JP H04 329939 A

### SUMMARY OF THE INVENTION

The invention is defined by the appended independent claim. Further embodiments of the invention are illustrated in the dependent claims.

### SUMMARY OF THE DISCLOSURE

### PROBLEMS TO BE SOLVED

In this type of the surgical tool, a slider included in the operation unit moves linearly; and this linear movement of the slider is transmitted to the treatment portion though the wire or the like. Accordingly, the treatment portion moves for a movement amount corresponding to the manipulation on the slider.

However, for example, when performing a delicate movement with the treatment portion, in other words, when a rather minute amount of operation is requested at the treatment portion, it is necessary to manipulate the slider for that amount of operation. This requires a delicate manipulation of the slider which have been difficult.

Patent Document 2 describes a traction device which can be used for medical instruments. The traction device comprises a deceleration pulley disposed in linear direction to a substrate which comprises a wrist sleeve. Further, the traction device comprises first and second reversing pulleys and primary and secondary cables.

Patent Document 3 describes an operating part of biopsy forceps which is equipped with a shaft-like member, and a slider which is connected to the shaft-like member in a freely slidable manner. An end part of a wire is introduced into the shaft-like member and a connector is attached to the wire. A recessed groove has a first rack formed at the bottom, and a protrusion which is to be engaged with the recessed groove is formed in the inner peripheral face of the slider and a second rack is formed on the tip part of the protrusion. A pinion is supported on the tip part of the connector in a freely rotating manner and is constantly engaged with the first rack and the second rack.

Patent Document 4 describes an endoscopic treatment instrument comprising an operation lever which can slide to the tip side of an inserting section, a pinion gear rotating with it, and a mobile rack moving to the tip side of the inserting part. With the movement of the mobile rack, a calculus recovery wire as treating part is inserted out of the tip of the inserting part. As the moving value of the mobile rack doubles the moving value of a pinion gear along a fixed rack, the moving value of the operation lever can be transmitted to a wire, being amplified.

An object of one aspect of the present disclosure is to provide a technique to improve usability of a surgical tool that includes an operation unit having linearly-moving sliders.

### MEANS FOR SOLVING THE PROBLEMS

One aspect of the present disclosure is a surgical tool including a slider, converters, and transmission wires. The slider is disposed movably in a linear direction relative to a main body. The main body includes at least a treatment portion for performing a medical procedure. The converters move for a converted movement amount which is obtained by converting the movement amount of the sliders with a specified magnification factor. The transmission wires transmit the converted movement amount to the treatment portion.

With such a configuration, the movement amount of the linearly-moving slider is converted by the converters with the specified magnification factor. Thus, the converted movement amount converted by the converters with the specified magnification factor is what is transmitted to the treatment portion. Accordingly, for example, by decreasing the specified magnification factor, the converted movement amount is reduced relative to the movement amount of the slider. As a result, the treatment portion moves in accordance with the converted movement amount, therefore, it becomes easier to perform a delicate work. In other words, it is possible to improve the usability of the surgical tool that includes an operation unit having a linearly-moving slider.

In one aspect of the present disclosure, each of the converters includes conversion wires, and movable pulleys. The conversion wires each have a first end attached to the sliders and a second end attached to the main body. The conversion wires are disposed along circumferential surfaces of the movable pulleys.

With such a configuration, the movement amount of the slider can be converted with the specified magnification factor by the movable pulleys and the conversion wires. Since it is possible to perform the conversion with the specified magnification factor by disposing the movable pulleys, it is not necessary to dispose an additional structure such as a lever. Therefore, it becomes easier to inhibit upsizing of the structure of the surgical tool due to the disposal of the structure such as the lever.

In one aspect of the present disclosure, the converters are disposed on one side and on an opposite side of moving directions of the slider.

With such a configuration, it is possible to transmit the converted movement amount, obtained by converting the movement amount of the slider with the specified magnification factor, to the treatment portion by using the converters disposed on one side and the opposite side of the moving directions of the slider.

In one aspect of the disclosure, the surgical tool comprises a plurality of sliders, and a plurality of converters which are each assigned to one of the plurality of sliders.

In one aspect of the disclosure, the surgical tool comprises a plurality of the movable pulleys, two of them assigned to one of the plurality of sliders, and when viewed from above, a linear direction connecting the centers of the two of movable pulleys and a moving direction of the plurality of sliders are parallel.

In one aspect of the disclosure, the surgical tool comprises a plurality of the movable pulleys, two of them assigned to one of the plurality of sliders, wherein centers of the two movable pulleys assigned to one slider are in straight line, and a longitudinal central axis of the assigned slider is on the same straight line.

In one aspect of the disclosure, the transmission wire has a first end attached to a rotating shaft of a movable pulley on a side closer to the treatment section, and a second end different from the first end which is attached to a rotating shaft of a movable pulley on the opposite side of the treatment section, thereby forming a loop for operating the treatment section.

### BRIEF DESCRIPTION OF THE DRAWINS

FIG. 1 is a diagram showing the whole of a surgical tool.
FIG. 2 is a diagram showing a structure of a movable portion.
FIG. 3 is a diagram showing a front surface of a main body.
FIG. 4 is a diagram showing a rear surface of the main body.
FIG. 5 is a diagram showing inside of the front surface of the main body.
FIG. 6 is a diagram schematically showing a configuration of a converter.

### EXPLANATION OF REFERENCE NUMERALS

1...surgical tool; 10...treatment portion; 11...movable portion 11A,11B...holding portion; 13...joint; 13A,13B...rotational shaft; 20...main body; 21...slider; 22... aperture; 23...movable pulley; 24...fixed pulley; 25...transmission wire; 27...conversion wire; 29...fixed portion; O...center axis.

### MODE FOR CARRYING OUT THE INVENTION

### [1. Configuration]

A surgical tool 1 in the present embodiment is a medical instrument used in medical practices such as surgeries.

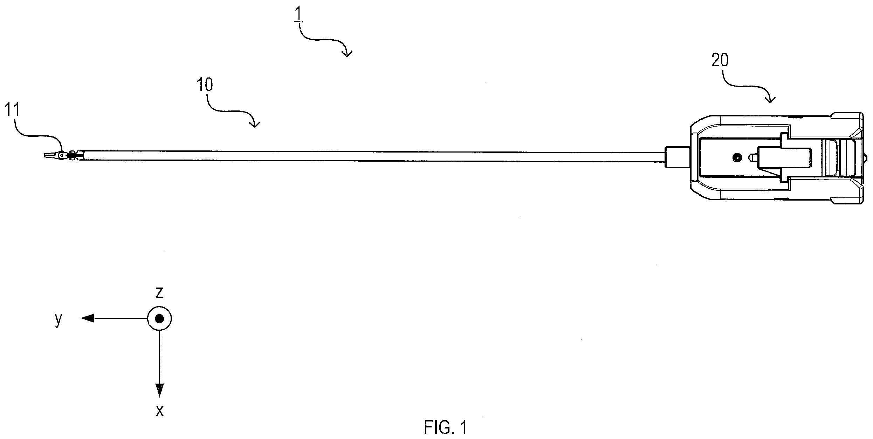

Structures of the surgical tool 1 will be explained with reference to FIG. 1 through FIG. 4.

As shown in FIG. 1, the surgical tool 1 includes a treatment portion 10, and a main body 20.

The treatment portion 10 is a part of the surgical tool 1 used for performing a treatment in a medical practice. For example, the treatment portion 10 is formed to have an elongated shape, and a first end of the elongated shape is coupled to the main body 20.

The treatment portion 10 includes a movable portion 11 at least on a part of the treatment portion 10.

The movable portion 11 is disposed on a leading end of the treatment portion 10, namely a second end, that is situated opposite the first end where the treatment portion 10 is coupled to the main body 20. The location where the movable portion 11 is situated is not limited to the end of the treatment portion 10 coupled to the main body 20 and the opposite end; the movable portion 11 may be situated on the treatment portion 10 at a location other than the said ends.

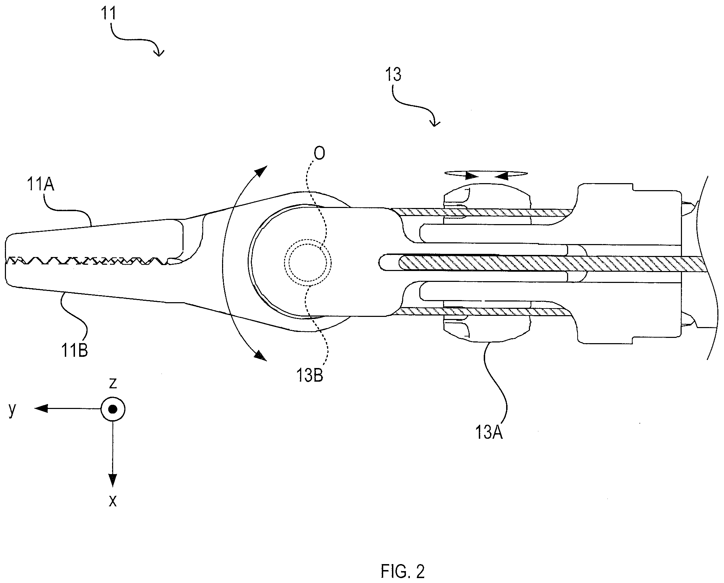

As shown in FIG. 2, the movable portion 11 has its head portion branched into two branches and is configured to be able to open and close the branches. The movable portion 11 is configured to be able to adjust the orientation of each branch so that the branches point in two different directions each perpendicular to the other branch. In other words, the movable portion 11 is displaceable (movable) relative to the treatment portion 10. The movable portion 11 may be used as a forceps, for example. In other words, the movable portion 11 may be used as a grasping unit of the treatment portion 10 capable of grasping a portion to be treated, a surgical needle, and the like. Each of the two branches, a holding portion 11A and a holding portion 11B, of the movable portion 11 is rotatable or pivotable about a center axis O between a closed position, in which the holding portion 11A and the holding portion 11B are situated close to each other, and an open position, in which they are situated apart from each other. Accordingly, the movable portion 11 is able to hold the portion to be treated and the like.

The open and close movement of the branches of the movable portion 11 may be caused by transmission of a drive force to the branched structure. A joint 13 that rotates in a direction to adjust the orientation of the head portion may be disposed; and the movement to adjust the orientation of the head portion may be caused by a structure in which the rotation of the joint 13 may adjust the orientation of the head portion of the movable portion 11. The joint 13 disposed in the movable portion 11 may include a first rotational shaft 13A in right-left directions (x-axis directions as mentioned later) and a second rotational shaft 13B in up-down directions (z-axis directions as mentioned later). In other words, the joint 13 is rotationally displaceable about the rotational shafts 13A and 13B as center axes. The first rotational shaft 13A is a shaft perpendicular to longitudinal directions of the treatment portion 10. The second rotational shaft 13B is a shaft perpendicular to the longitudinal directions of the treatment portion 10 and to the first rotational shaft 13A.

the movable portion 11 is formed to have a structure that responds to an operation of the treatment portion 10. The movable portion 11 is not limited to having both a structure that enables holding an object and releasing the object and a structure to rotate the head portion to adjust the orientation of the head portion. The movable portion 11 may include either one of a structure that enables holding an object and releasing the object or a structure to rotate the head portion to adjust the orientation of the head portion. The movable portion 11 may also be configured to be able to perform a different movement.

Elements and parts necessary to cause the treatment portion 10 to operate are arranged in the main body 20.

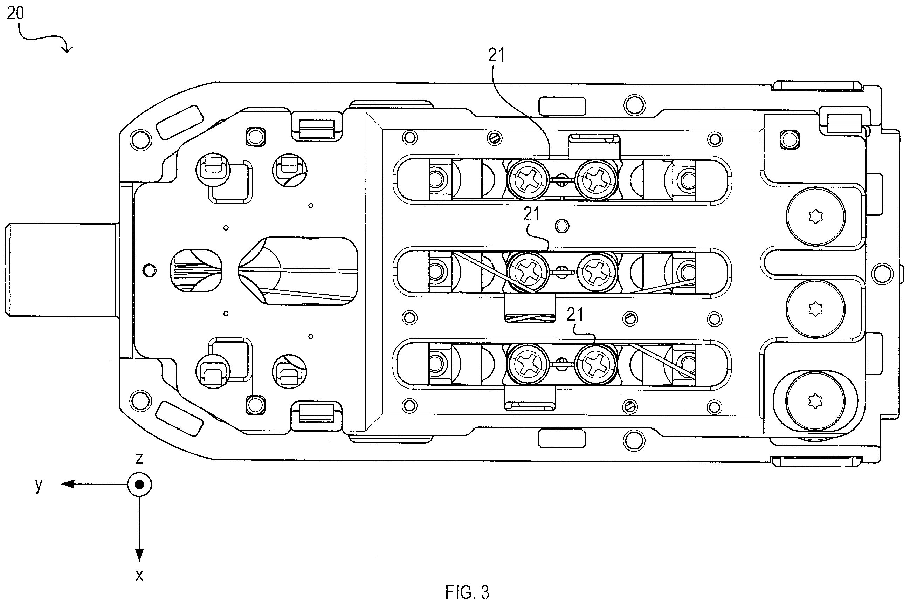

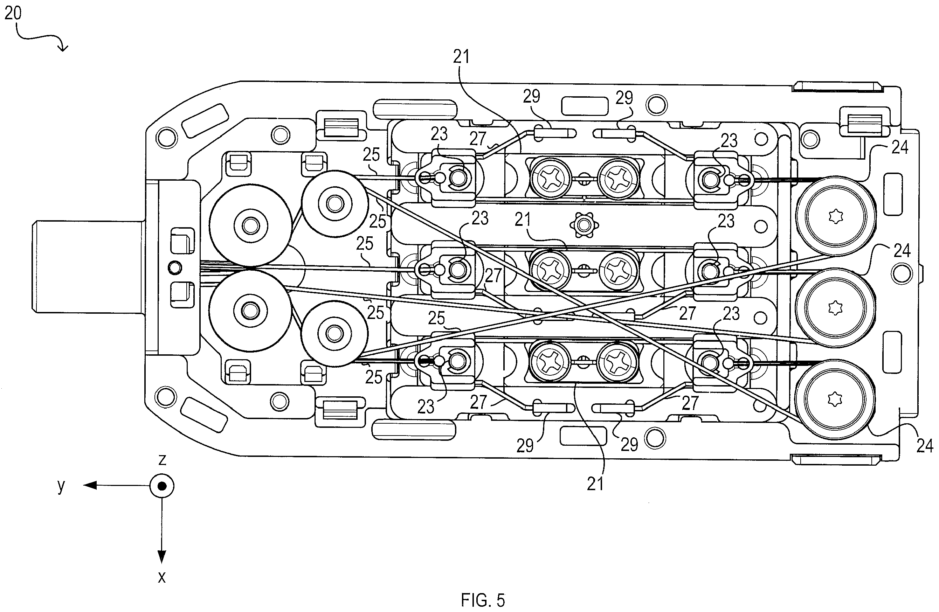

As shown in FIG. 3, FIG. 4, and FIG. 5, sliders 21, apertures 22, movable pulleys 23, transmission wires 25, conversion wires 27, and fixed portions 29 are arranged in the main body 20.

Up-down directions of the main body 20 are also referred to as the z-axis directions. The upward direction is also referred to as a z-axis positive direction; the downward direction is also referred to as a z-axis negative direction. In addition, long-side directions of a rectangle of an opening of the main body 20, in other words, the longitudinal directions of the treatment portion 10 coupled to the main body 20, are referred to as y-axis directions. When viewing from the main body 20, a direction where the treatment portion 10 is positioned is also referred to as a y-axis positive direction; the opposite direction is also referred to as a y-axis negative direction. In addition, an axis perpendicular to a y-z plane is referred to as an x-axis. When facing the y-axis positive direction and having the z-axis positive direction as an upper direction, the left side is also referred to as an x-axis positive direction, and the right side is also referred to as an x-axis negative direction. The x-axis directions are also referred to as right-left directions, and z-axis directions are also referred to as up-down directions. In addition, a surface of the main body 20 that can be seen when viewing the main body 20 from the z-axis positive side to the z-axis negative side is also referred to as a front surface. A surface of the main body 20 that can be seen when viewing the main body 20 from the z-axis negative side to the z-axis positive side is also referred to as a rear surface.

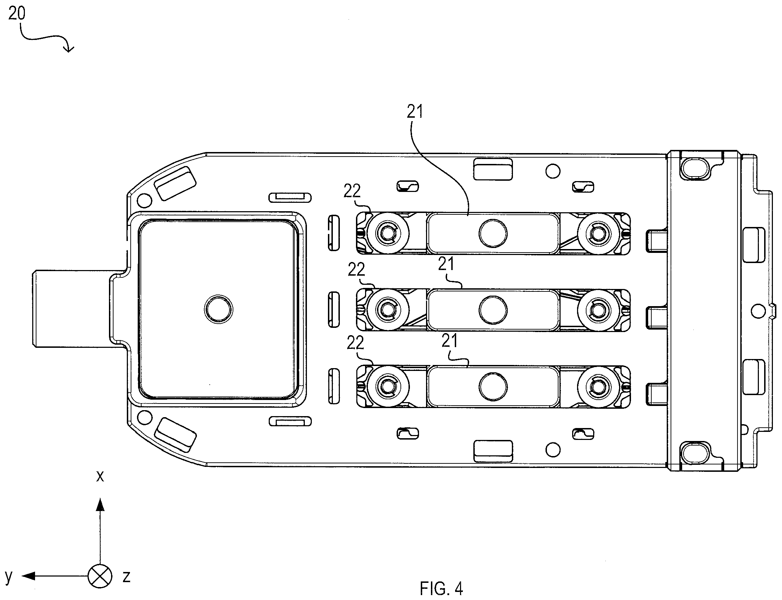

As shown in FIG. 4, the main body 20 includes apertures 22 on the rear surface. The apertures 22 are rectangular through holes. The number of such rectangular apertures matches with the number of the sliders 21. As shown in the drawings from FIG. 3 through FIG. 5, the number of the sliders 21 included in the main body 20 is three in the present embodiment. The number of the apertures 22 is also three. The number of the sliders 21 and the number of apertures should not be limited to three; they may be more than three or less than three. Each aperture 22 is arranged such that its long side direction of the rectangular aligns with the longitudinal direction of the elongated treatment portion 10.

The sliders 21 are each arranged inside the apertures 22 formed in the main body 20 such that each slider 21 is movable along the longitudinal direction of the aperture 22. By making a reciprocating movement, each slider 21 generates a drive force for displacing the movable portion 11.

Each slider 21 is formed into a rectangular solid and arranged such that the long side of the rectangular solid aligns with the longitudinal direction of the rectangular (aperture). At least a part of surfaces of each slider 21 exposes from an upper surface and a lower surface of the main body 20.

An end portion of the conversion wire 27 is fixed to an end of each slider 21 in the y-axis directions.

The other end portion of the conversion wire 27, opposite the end portion fixed to the slider 21, includes a fixed portion 29 that is to be fixed to the main body 20. The fixed portion 29 is fixed to the main body 20. The location to dispose the fixed portion 29 is not particularly limited; however, the fixed portion 29 may be disposed, for example, in an area of the main body 20 adjacent the aperture 22 and close to the center in the x-axis directions.

One movable pulley 23 is disposed for one conversion wire 27. The movable pulley 23 is arranged such that one conversion wire 27 is disposed along a circumferential surface of the movable pulley 23. One slider 21 is disposed between two movable pulleys 23. The movable pulley 23 is disposed such that its rotational shaft extends in the up-down directions, in other words, in the z-axis directions.

An element that holds the rotational shaft of the movable pulley 23 is coupled to the transmission wire 25. The transmission wire 25 is configured to transmit the drive force to move the treatment portion 10 and the movable portion 11. The transmission wire 25 is not limited to being coupled to the element that holds the rotational shaft of the movable pulley 23. The transmission wire 25 may also be coupled directly to the rotational shaft of the movable pulley 23. In other words, the transmission of the drive force may be achieved directly or indirectly as long as it is configured such that the drive force can be transmitted between the rotational shaft of the movable pulley 23 and the treatment portion 10 and the movable portion 11.

A first end of the transmission wire 25 is attached to the rotational shaft of the movable pulley 23 situated close to the treatment portion 10. A second end of the transmission wire 25, which is different from the first end of the transmission wire 25, is attached to the rotational shaft of the movable pulley 23 situated away from the treatment portion 10. The transmission wire 25 extends towards the treatment portion 10 and the movable portion 11 from the movable pulley 23 situated close to the treatment portion 10. The transmission wire 25 returns at the treatment portion 10 and the movable portion 11. The transmission wire 25 returned at the treatment portion 10 and the movable portion 11 then returns along a fixed pulley 24 that is fixed to the main body 20. The transmission wire 25 returned along the fixed pulley 24 is then attached to the rotational shaft of the movable pulley 23 situated opposite the treatment portion 10 relative to the slider 21. Displacement of the slider 21 is transmitted to the transmission wire 25 through the movable pulleys 23 and the fixed pulley 24.

The transmission wires 25 are directly or indirectly coupled to the holding portion 11A and the holding portion 11B, which are branched, and to the joint 13. The transmission wires 25 are not limited to being directly or indirectly coupled to the holding portion 11A and the holding portion 11B, which are branched, and to the joint 13 as long as the drive force for making a movement is transmitted to the movable portion 11.

The transmission wire 25 coupled to one of the sliders 21 may be coupled to a holding structure; and the transmission wire 25 coupled to one of the sliders 21 may be arranged on a circumferential surface of a rotating portion of the joint 13, which is included in the movable portion 11 and has a structure to adjust the orientation of the movable portion 11.

The movable pulley 23 and the conversion wire 27 of the present embodiment correspond to one example of a configuration as a converter.

### [2. Movement (Operation)]

Movement of the sliders 21, the movable pulleys 23, and the transmission wires 25 in addition to the movement of the movable portion 11 will be explained.

In response to a manipulation by an operator, each slider 21 linearly moves inside the aperture 22 in the longitudinal directions of its rectangular shape.

The conversion wire 27, attached to the end of the slider 21, simultaneously moves with the linear movement of the slider 21. This causes the movable pulley 23, on the circumferential surface of which the conversion wire 27 is arranged, to move. Then, by the movement (displacement) of the first end of the transmission wire 25 coupled to the rotational shaft of the movable pulley 23, the drive force is transmitted to the second end of the transmission wire 25 coupled to the movable portion 11. The transmission wire 25 moves in accordance with a movement amount of the linear movement of the slider 21. The movement amount of the transmission wire 25 is also referred to as a converted movement amount.

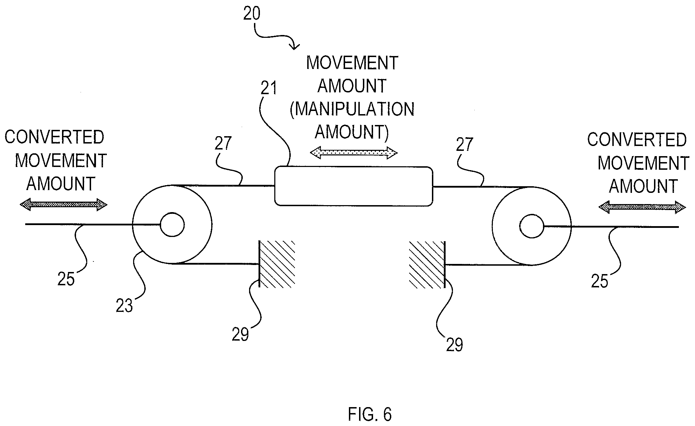

The converted movement amount changes in accordance with the number of movable pulleys 23 that move in conformity with the movement of the sliders 21. Specifically, as shown in FIG. 6, in a case where the number of movable pulleys 23 that moves in accordance with the movement amount of the sliders 21, in other words, an amount of manipulation on the sliders 21 by the operator, is two, the movement amount will be one half compared with a case where fixed pulleys are used in place of the movable pulleys 23. A force twice as much as the force to manipulate the slider 21 is applied to the transmission wire 25 as the drive force.

Due to the linear movement of the sliders 21, the drive force applied to the transmission wire 25 and the converted movement amount are transmitted to the movable portion 11. The movable portion 11 moves in accordance with the transmitted converted movement amount of the transmission wire 25 and the transmitted drive force.

In the present embodiment, the movement of the movable portion 11 in accordance with the transmitted converted movement amount of the transmission wire 25 and the transmitted drive force is, for example, a holding movement and a release movement by the holding portion 11A and the holding portion 11B, and a rotational movement of the joint 13. In other words, the holding movement and the release movement may be made by the holding portion 11A and the holding portion 11B approaching or separating from each other about the center axis O in response to the movement of the transmission wire 25 that directly or indirectly transmits the drive force and the converted movement amount to the holding portion 11A and the holding portion 11B. The movable portion 11 may be configured to accordingly open the holding structure at its head portion for the converted movement amount.

In addition, the joint 13 may make a rotational displacement movement about the rotational shaft 13A and the rotational shaft 13B in response to the movement of the transmission wire 25 that directly or indirectly transmits the drive force and the converted movement amount to the joint 13. The orientation of the head portion of the movable portion 11 may be configured to be adjusted accordingly for the converted movement amount.

### [3. Effects]

(1) In the present embodiment, the movement amount of the linear movement of the slider 21 is converted to the converted movement amount by the movable pulleys 23 and the conversion wires 27, and the transmission wire 25 moves for the converted movement amount. The movement amount of the transmission wire 25 is transmitted to the movable portion 11, and the movable portion 11 moves in accordance with the movement amount of the transmission wire 25.

By increasing the number of the movable pulleys 23 and the conversion wire 27 that receives the transmission of the movement amount of the slider 21, the converted movement amount of the transmission wire 25 relative to the movement amount of the slider 21 is reduced, and accordingly, the converted movement amount transmitted to the movable portion 11 is reduced.

Consequently, it becomes easy to perform a detailed work since the movable portion 11 of the treatment portion 10 moves in accordance with the converted movement amount. In other words, usability of the surgical tool 1 that includes an operation unit having the linearly-moving sliders 21 can be improved.

(2) Furthermore, by changing the number of the movable pulleys 23 and the conversion wires 27, the converted movement amount of the transmission wire 25 relative to the movement amount of the linear movement of the slider 21 can be changed. In other words, the converted movement amount of the transmission wire 25 relative to the movement amount of the sliders 21 can be changed depending on the number of the fixed pulleys changed to the movable pulleys 23. Accordingly, it is not necessary to dispose a structure such as a lever to change the movement amount of the transmission wire 25. Therefore, upsizing of the main body 20 can be inhibited compared with a case where an element such as a lever is disposed.

### [Modifications of Embodiment]

(1) In the aforementioned embodiment, the number of the slider 21 is two or more. However, the number of the slider 21 is not limited to a plural number and may be one.
(2) In the aforementioned embodiment, an example was provided in which two movable pulleys 23 moves for one slider 21. However, the number of the movable pulleys 23 that moves for the slider 21 is not limited to two. For example, the number of the movable pulleys 23 that moves for the slider 21 may be greater or less than two.
   As the number of the movable pulleys 23 that moves relative to the number of the slider 21 increases, the movement amount of each movable pulley 23 relative to the movement amount of the slider 21 decreases. Accordingly, it is possible to adjust the amount of operation of the movable portion 11 relative to the amount of manipulation on the slider 21 by adjusting the number of movable pulleys 23 that is coupled to the slider 21.
(3) Each of the transmission wires 25 may be coupled to different part of the movable portion 11.

In response to a movement of each of the transmission wires 25 coupled to different parts of the movable portion 11, the movable portion 11 may move differently in accordance with the moved transmission wire 25.

Specifically, for example, two holding portions respectively holding the yz-plane and xy-plane may be disposed, and the transmission wire 25 for transmitting the drive force may be coupled to each of the holding portions such that the orientation (direction) to make the holding movement and the orientation (direction) to make the releasing movement are different.

In addition, the movement of the movable portion 11 is not limited to the holding movement. The movable portion 11 may be formed to make a rotational movement to be able to adjust the orientation of the head portion of the movable portion 11.

It may be configured such that the holding movement is achieved by manipulating one of the sliders 21 corresponding to one of the transmission wires 25 and such that the rotational movement of the head portion is achieved by manipulating another one of the sliders 21 corresponding to another one of the transmission wires 25.

(4) In the aforementioned embodiment, the movable pulleys 23 moves in accordance with the movement of the corresponding sliders 21. However, the movable pulleys 23 are not limited to those that move in accordance with the movement of the sliders 21 and may be configured to be changeable between a fixed state and a movable state.

(5) In the aforementioned embodiment, the exposed parts of the sliders 21 is configured to be manipulated by the operator and displaced. However, the sliders 21 are not limited to those whose exposed parts are directly manipulated by the operator. For example, each slider 21 may be displaced by receiving a drive force from a pneumatic cylinder and the like.

## Claims

1. A surgical tool (1) comprising:
a slider (21) disposed movably in a linear direction relative to a main body (20), the main body (20) including at least a treatment portion (10) configured to perform a medical procedure;
converters configured to move for a converted movement amount which is obtained by converting a movement amount of the slider (21) with a specified magnification factor; and
wherein each of the converters comprises:
conversion wires (27) each having a first end attached to the slider (21) and a second end attached to the main body (20); and
movable pulleys (23), wherein the conversion wires (27) are disposed along circumferential surfaces of the movable pulleys (23), and
wherein the converters are disposed on one side and on an opposite side of moving directions of the slider (21), and
transmission wires (25) coupled to the movable pulleys by an element that holds the rotational shaft of the movable pulleys (23) and configured to transmit the converted movement amount to the treatment portion (10).

2. The surgical tool (1) according to claim 1, comprising a plurality of sliders (21), a plurality of converters which are each assigned to one of the plurality of sliders (21).

3. The surgical tool (1) according to claim 1, wherein the transmission wire (25) has a first end attached to a rotating shaft of a movable pulley (23) on a side closer to the treatment section (10), and a second end different from the first end which is attached to a rotating shaft of a movable pulley (23) on the opposite side of the treatment section (10), thereby forming a loop for operating the treatment section (10).

## Patentansprüche

1. Chirurgisches Werkzeug (1), enthaltend:
ein Schieber (21), der in einer linearen Richtung relativ zu einem Hauptkörper (20) beweglich angeordnet ist, wobei der Hauptkörper (20) mindestens einen Behandlungsabschnitt (10) einschließt, der zum Durchführen eines medizinischen Eingriffs konfiguriert ist;
Konverter, die konfiguriert sind, sich um einen konvertierten Bewegungsbetrag zu bewegen, der durch Konvertieren eines Bewegungsbetrags des Schiebereglers (21) mit einem bestimmten Vergrößerungsfaktor erhalten wird; und
wobei jeder der Konverter enthält:
Konversionsdrähte (27), die jeweils ein erstes, an dem Schieber (21) befestigtes Ende und ein zweites, an dem Hauptkörper (20) befestigtes Ende aufweisen; und
bewegliche Riemenscheiben (23), wobei die Konversionsdrähte (27) entlang umlaufender Oberflächen der beweglichen Riemenscheiben (23) angeordnet sind, und
wobei die Konverter auf einer Seite und auf einer gegenüberliegenden Seite der Bewegungsrichtungen des Schiebers (21) angeordnet sind; und
Übertragungsdrähte (25), die mit den beweglichen Riemenscheiben durch ein Element gekoppelt sind, das die Rotationswelle der beweglichen Riemenscheiben (23) hält, und die konfiguriert sind, den konvertierten Bewegungsbetrag zu dem Behandlungsabschnitt (10) zu übertragen.

2. Chirurgisches Werkzeug (1) nach Anspruch 1, enthaltend eine Vielzahl von Schiebern (21), eine Vielzahl von Konvertern, die jeweils einem der Vielzahl von Schiebern (21) zugeordnet sind.

3. Chirurgisches Werkzeug (1) nach Anspruch 1, wobei der Übertragungsdraht (25) ein erstes Ende, das an einer Rotationswelle einer beweglichen Riemenscheibe (23) auf einer Seite befestigt ist, die dem Behandlungsabschnitt (10) näher liegt, und ein zweites, von dem ersten Ende verschiedenes Ende aufweist, das an einer Rotationswelle einer beweglichen Riemenscheibe (23) auf der gegenüberliegenden Seite des Behandlungsabschnitts (10) befestigt ist, dadurch eine Schlaufe zum Betätigen des Behandlungsabschnitts (10) bildend.

## Revendications

1. Un outil chirurgical (1) comprenant :
un coulisseau (21) disposé de manière mobile dans une direction linéaire par rapport à un corps principal (20), le corps principal (20) incluant au moins une partie de traitement (10) configurée pour effectuer une procédure médicale ;
des convertisseurs configurés pour se déplacer pour une quantité de mouvement convertie qui est obtenue en convertissant une quantité de mouvement du coulisseau (21) avec un facteur de grossissement spécifié ; et
dans lequel chacun des convertisseurs comprend :
des fils de conversion (27) ayant chacun une première extrémité fixée au coulisseau (21) et une seconde extrémité attachée au corps principal (20) ; et
des poulies mobiles (23), dans lequel les fils de conversion (27) sont disposés le long des surfaces circonférentielles des poulies mobiles (23), et
dans lequel les convertisseurs sont disposés d'un côté et d'un côté opposé des directions de déplacement du coulisseau (21), et
des fils de transmission (25) couplés aux poulies mobiles par un élément qui tient l'arbre rotatif des poulies mobiles (23) et configurés pour transmettre la quantité de mouvement convertie à la partie de traitement (10).

2. L'outil chirurgical (1) selon la revendication 1, comprenant une pluralité de coulisseaux (21), une pluralité de convertisseurs qui sont chacun affectés à l'un de la pluralité de coulisseaux (21).

3. L'outil chirurgical (1) selon la revendication 1, dans lequel le fil de transmission (25) a une première extrémité fixée à un arbre rotatif d'une poulie mobile (23) sur un côté plus proche de la section de traitement (10), et une seconde extrémité différente de la première extrémité, qui est fixée à un arbre rotatif d'une poulie mobile (23) sur le côté opposé de la section de traitement (10), formant ainsi une boucle pour le fonctionnement de la section de traitement (10).
